## Europäisches Patentamt
## European Patent Office
## Office européen des brevets

(19) ■))

(11) Numéro de publication: **0 135 436**
**B1**

(12) **FASCICULE DE BREVET EUROPÉEN**

(45) Date de publication du fascicule du brevet:
**13.08.86**

(21) Numéro de dépôt: **84401700.4**

(22) Date de dépôt: **22.08.84**

(51) Int. Cl.⁴: **C 07 C 11/02,** C 07 C 1/253,
C 07 C 1/36, B 01 J 23/82

(54) **Nouveau procédé de fabrication d'oléfine linéaire à partir d'acide gras ou d'ester d'acide gras saturé.**

(30) Priorité: **25.08.83 FR 8313822**

(43) Date de publication de la demande:
**27.03.85 Bulletin 85/13**

(45) Mention de la délivrance du brevet:
**13.08.86 Bulletin 86/33**

(84) Etats contractants désignés:
**BE DE GB**

(56) Documents cité:
**EP-A-0 000 054**

**CHEMISCHE BERICHTE, vol. 115, no. 2, 1982, pages 808-812, Verlag Chemie GmbH, Weinheim, DE; W.F. MAIER et al.: "Gas phase decarboxylation of carboxylic acids"**

(73) Titulaire: **INSTITUT FRANCAIS DU PETROLE, 4, Avenue de Bois- Préau, F-92502 Rueil- Malmaison (FR)**

(72) Inventeur: **Stern, Robert, 5, rue Nollet, F-75017 Paris (FR)**
Inventeur: **Hillion, Gérard, 10, Place du Cassan, F-95220 Herblay (FR)**

## Description

Il est connu de fabriquer des oléfines à partir d'acides gras saturés en utilisant des métaux nobles en présence de phosphine comme catalyseurs. Ainsi, Foglia et Barr JAOCS *53*, 737 (1976) décarbonylent à 280° l'acide stéarique avec des complexes phosphinés du rhodium et un excès de phosphine du type triphenylphosphine. Ils obtiennent ainsi l'heptadécène.

De même, Fenton US 3530 198 obtient une oléfine en partant d'acides gras et en opérant avec du chlorure de palladium en présence de chlorure de lithium et de phosphines variées.

Ces procédés catalytiques ne semblent pas à priori économiquement valables pour deux raisons: la première est le coût de l'investissement un catayseur, la seconde est le manque de stabilité des catalyseurs.

Dans le brevet US 4,102,938 une huile végétale est pyrolisée en présence d'oxydes mixtes avec des métaux très divers. Le mélange complexe contient des oléfines mais aussi des hydrocarbures saturés, des cétones, parfois des aromatiques et surtout des produits de cracking. Il est pratiquement impossible de sortir une oléfine de ce mélange.

Si l'on considère plus spécialement les catalyseurs au nickel, on constate qu'ils ont tendance à donner à certaines températures supérieures à 200°C des produits de cracking ou bien, comme l'indique W. F. Maier, Chem. Ber. *115*, 808—812 (1982) des hydrocarbures saturés, par décarboxylation à 180° à pression atmosphérique. Ainsi, en faisant passer en phase gazeuse l'acide octoïque sur un catalyseur au nickel en présence d'hydrogène, on obtient 64% d'heptane qui n'est pas un produit à grande valeur ajoutée.

Divers auteurs ont encore chauffé l'acide stéarique avec du nickel de Raney et ont obtenu un mélange de gaz contenant du CO et surtout du $CO_2$, un acide éthylénique, de la stéarone, du stéarate de nickel et 15% d'hydrocarbures éthyléniques non spécifiés. La plus grande partie du nickel s'est retrouvée sous la forme de stéarate de nickel après 200 heures à 200—230°.

Ainsi, le nickel semble être un métal capable soit de catalyser la fabrication d'hydrocarbures saturés, soit d'induire la formation de mélanges complexes tout en se consommant.

Il n'existe donc, apparemment, aucun procédé permettant avec un métal non noble d'obtenir sélectivement des oléfines linéaires à partir d'acides ou d'esters carboxyliques linéaires.

Il a été trouvé de manière surprenante qu'un système nickel-étain, nickel-germanium et/ou nickel-plomb, permettait d'obtenir, de préférence en présence d'hydrogène, à partir d'un acide ou d'un ester carboxylique, des oléfines avec une sélectivité remarquable. Les oléfines ont un atome de carbone de moins que l'acide ou que la partie acide de l'ester. On récupère dans les fractions de tête après distillation un alcool correspondant à l'alcool utilisé dans l'ester, si l'acool est assez stable, ou de l'eau si l'on est parti d'un acide.

La teneur en produits de cracking indésirables est très faible, parfois nulle. On peut donc attendre dans les meilleurs cas un rendement très proche du rendement théorique en oléfine.

Ceci constitue donc une nouvelle voie de fabrication économique d'oléfines linéaires non branchées dont la double liaison est située majoritairement en position 1, avec une proportion variable d'oléfines dont la double liaison est en une autre position, essentiellement 2, 3 et/ou 4. Cette voie peut être mise en concurrence avec la fabrication d'oléfines linéaires à partir d'éthylène par oligomérisation puisque le prix de l'éthylène est à peine moins élevé que celui de certaines huiles ou graisses végétales ou animales saturées.

Cette réaction s'applique en particulier aux acides carboxyliques linéaires saturés ayant de 6 à 30 atomes de carbone ainsi qu'aux esters formés entre ces acides et des alcools mono ou polyhydriques, par exemple les monoalcools ayant de 1 à 4 atomes de carbone, les glycols et le glycérol, ainsi qu'aux esters partiels du glycérol. Comme exemples, on peut citer les huiles saturées qui sont des esters de la glycérine et d'acides gras saturés, mais de préférence les esters d'alcools inférieurs, par exemple les esters méthylique, éthylique, propylique ou butylique des acides gras tirés du suif, de l'huile de palme, de la fraction solide du palme, de l'huile de coprah, de babassu ou de palmiste, ou obtenus par hydrogénation totale d'huile ou de graisse ou des esters des huiles insaturées. Les esters d'alcools inférieurs peuvent être obtenus par exemple par transestérification des huiles ou graisses ou par estérification des acides gras correspondants. Parmi les acides gras saturés on peut mentionner ceux obtenus par cristallisation fractionnée des acides gras de suif.

L'objet de l'invention est spécifiquement l'obtention d'oléfines avec un catalyseur constitué par du nickel et un métal du groupe étain, germanium et/ou plomb et plus particulièrement l'utilisation du système nickel-étain.

Le système catalytique précité présente des propriétés originales puisque d'autres couples testés sont inertes ou donnent principalement des produits de cracking ou de décarboxylation. La présence d'hydrogène est nécessaire pour maintenir l'activité du catalyseur. Mais il est possible d'utiliser un gaz inerte pendant un certain temps. Les conditions de la réaction sont relativement larges puisque la gamme de température est de 200 à 400°C, mais de préférence 300 à 380°C. La pression adoptée peut être la pression atmosphérique ou être supérieure à 1 bar. En particulier, lorsque les acides ou les esters sont trop volatils, il est intéressant de travailler sous pression super atmosphérique pour rester en phase liquide. Toutefois, on peut réaliser la réaction aussi bien en phase gazeuse qu'en phase liquide, en discontinu ou continu. Les VVH sont de préférence de 0,1 à 10 vol/vol/heure si l'on considère la phase liquide.

2

Einfin, on peut ajouter au catalyseur précité des additifs comme par exemple des dérivés soufrés pour diminuer la capacité hydrogénante du nickel et rendre la réaction encore plus sélective. D'autres additifs utilisables sont à base d'arsenic ou de phosphore.

Le catalyseur peut être préparé par addition d'un composé d'étain, de germanium ou de plomb à du nickel de Raney ou à un nickel supporté. Les supports de nickel sont de préférence non acides, comme par exemple des silices à basse et haute surface, l'oxyde de titane, l'oxyde de zinc et certaines alumines à basse surface ou alcalinisées. Quand le nickel est supporté, sa teneur est, par exemple, de 1 à 50% en poids de l'ensemble Ni+support.

Comme composé de l'étain, on peut signaler les halogénures d'étain bivalents ou tétravalents, les stannates de métaux alcalins ou alcalino-terreux, le stannate de nickel, mais de préférence des dérivés hydrocarbyle d'étain. Comme exemples de composés hydrocarbyles d'étain, on peut citer le tétraméthyl étain, le tétrabutyl étain, le tétrapropyl étain, le tétraphényl, étain, le dibutyl dilaurate d'étain. Comme exemples de composés du plomb, on peut citer le tétraéthyl plomb, le tétraméthyl plomb, l'acétate de triméthyl plomb, l'acétate de triéthyl plomb, le tétraphényl plomb et le tétrabutyl plomb ou des chlorures de plomb, silicate ou carboxylate de plomb. Comme exemples de composés du germanium, citons des chlorures de germanium, le tétraméthyl, le tétraphényl et le tétrabutyl germanium.

Le rapport pondéral (Sn+Ge+Pb)/Ni est utilement compris entre 0,001:1 et 10:1 et de préférence entre 0,05:1 et 1:1.

Pour fabriquer le catalyseur, on opère de préférence en imprégnant le nickel solide (nickel Raney ou nickel supporté) par un composé d'étain, de germanium ou de plomb, de préférence à l'ébullition dans un solvant, ou en imprégnant un support par une solution commune des métaux ou par des solutions distinctes du composé de nickel et du composé de métal additionnel. On peut également faire passer une solution d'hydrocarbyl-étain, -plomb ou -germanium dans une colonne contenant le nickel sous forme réduite. Si le composé de métal additionnel est liquide, un solvant n'est pas indispensable. Comme solvant on peut utiliser, par exemple, l'eau si le composé de métal n'est pas décomposé par cette dernière. Avec les composés hydrocarbyl on utilise on milieu anhydre, par exemple un milieu hydrocarbure. Si le métal additionnel est sous forme d'un hydrocarbyl métal, il n'est pas nécessaire de procéder à d'autres opérations après l'imprégnation, si ce n'est parfois in lavage aux hydrocarbures du composé qui n'a pas réagi. S'il s'agit d'un métal additionnel ajouté sous forme ionisée, il est préférable de chauffer à haute température pour le réduire sous courant d'hydrogène, par exemple à 400—500°C. Dans certains cas, on peut réduire simultanément le nickel et le composé additionnel; la réduction peut se faire à l'hydrogène ou avec des agents réducteurs du type alkyl aluminium, les hydrures de sodium ou d'autres agents réducteurs chimiques connus.

En dernier lieu, on peut imprégner le catalyseur avec un inhibiteur d'hydrogénation. Ce dernier composé peut être un dérivé soufré. Les composés soufrés sont par exemple les mercaptans, les disulfures, le thiophène ou l'hydrogène sulfuré. Le dérivé soufré peut être introduit dans la charge au cours de la réaction. Comme les dérivés soufrés sont souvent volatils aux températures où la réaction est pratiquée, on peut imprégner préalablement le catalyseur et ajouter en continu des traces de dérivés soufrés.

Les quantités de soufre introduites correspondent avantageusement à 0,001 à 5% en poids du nickel, de préférence 1 à 3% en poids du nickel.

A la place des dérivés soufrés, on peut introduire des déivés du phosphore comme les phosphines ou des dérivés de l'arsenic. Parmi les composés du phosphore, citons entre autres les phosphines aromatiques.

Si la conversion de l'ester ou de l'acide n'est pas totale, il est facile d'isoler les oléfines par distillation en recyclant le résidu de distillation qui contient l'acide ou l'ester qui n'a pas réagi.

L'oléfine obtenue est un bon substrat pour l'hydroformylation ou la formation d'alkyl aromatiques.

Les exemples suivants illustrent l'invention. CPV est mis pour chromatographie en phase vapeur.

**Exemple 1**

Dans un ballon muni d'un dispositif de distillation on introduit successivement 50 g d'acide palmitique, 10 g de nickel de Raney sec dans du cyclohexane et 870 mg de tétrabutyl étain. On chauffe lentement en distillant le cyclohexane et l'on continue en faisant passer un courant d'hydrogène, le ballon étant amené à une température de 300°—310°. Un produit liquide distille entre 210 et 230°. De temps en temps, on injecte des quantités supplémentaires d'acide palmitique, en tout 150 g, dans le ballon, tout en distillant au fur et à mesure un produit, qui après analyse en CPV s'avère être principalement un mélange d'isomères de position pentadécène linéaire comprenant environ 60% de pentadécène-1. Il s'est formé un peu de pentadécane. Le poids d'oléfine en $C_{15}$ est de 63,5 g; la sélectivité en oléfine $C_{15}$ est de 69% par rapport à l'acide converti.

**Exemple 2 (comparaison)**

On chauffe 10 g de nickel de Raney avec 50 g d'acide palmitique et l'on opère comme dans l'exemple 1, excepté que l'étain est absent. On recueille, après injection de 83 g d'acide palmitique total, 46,7 g de liquide distillé qui ne contient que 3,5 g de pentadécène, la mejeure partie étant des produits de cracking. La sélectivité par rapport à l'acide converti est de 6%.

On voit donc que l'introduction de l'étain a décuplé le rendement en oléfine.

**0 135 436**

Exemple 3

50 g d'acide palmitique et 10 g d'un catalyseur contenant 25% de nickel sur silice sont chauffés avec 217 mg de tétrabutyl étain. Au lieu de faire passer de l'hydrogène, on passe de l'argon à raison de 100 cm³/min. Entre 280 et 320°, il distille 32 g d'un liquide peu acide (9,3% d'acide palmitique). Ce produit est constitué principalement de pentadécène linéaire et de pentadécane. La sélectivité en pentadécène est de 71%.

Exemple 4

Pour montrer la spécificité du nickel et en particulier du couple nickel-étain, divers catalyseurs ont été testés.

Les résultats, en partant de 100 g d'acide palmitique et en opérant comme dans l'exemple 1, sont présentés dans le tableau suivant:

| Catalyseur | Produit distillé en g | Sélectivité d'oléf. en $C_{15}$ | Remarque |
|---|---|---|---|
| Cobalt de Raney (10 g)+tétrabutyl étain (0,87 g) | mousse | 2% | palmitate de cobalt, cracking |
| Fer de Raney (10 g)+tétrabutyl étain (0,87 g) | mousse abondante | ≤0,5% | cétone, décarboxylation totale |
| Chromite de cuivre (10 g) | 35 | 1—2% | peu actif, cracking |
| Palladium sur charbon à 5% 10 g | 30 | traces | peu actif, cracking en hydrocarbure saturé |
| Nickel-étain 10 g (Catalyseur de l'exemple 1) | 71 | 65% | Pas de cracking |

Exemple 5

On utilise dans cet essai le catalyseur de l'exemple 1 avec pour substrat l'ester éthylique de palme hydrogéné. Il se forme principalement du pentadécène et de l'heptadécène linéaires faiblement isomérisés. Le rendement molaire en oléfine est de 49% par rapport à l'ester total et la sélectivité de 71%.

Exemple 6 (comparaison)

Le même essai que dans l'exemple 2 est réalisé avec l'ester éthylique de palme hydrogéné. Le rendement n'est plus que de 3% en un mélange de pentadécène et heptadécène linéaires et la sélectivité inférieure à 5%.

Exemple 7

On réalise le même essai qu'à l'exemple 1 mais avec un mélange des esters éthyliques d'acides palmitique et stéarique et en ajoutant en plus au catalyseur 1% de dibutyl sulfure par rapport au nickel. On obtient des oléfines linéaires en $C_{15}$ et $C_{17}$ avec un rendement en oléfine de 47%, un rapport oléfine/saturé >100 et une sélectivité par rapport à l'ester converti de 85%.

Exemple 8

On injecte en continu à 370° un ester méthylique de l'acide stéarique dans un tube contenant un catalyseur au nickel sur silice préalablement imprégné de tétrabutyl étain comme dans l'exemple 3 et de dibutyl sulfure comme dans l'exemple 7.

A VVH=0,66 on obtient une conversion molaire de 72% de l'ester en oléfine linéaire. L'oléfine ne contient pas de produit de cracking.

Le bilan s'établit ainsi:

. Conversion de l'ester 83%
. Heptadécène par rapport à l'ester converti: 85,5%
. Heptadécane par rapport à l'ester converti: 11%
. Cétones en $C_{31}$, $C_{33}$ et $C_{35}$ par rapport à l'ester converti: 3%

4

Exemple 9

Le même essai que dans l'exemple 8 est réalisé avec le laurate d'éthyle. Le rapport oléfine/saturé est de 17. La conversion est toutefois faible: 21% de l'ester a été converti en une oléfine linéaire très pure facilement distillée et séparée de l'ester non converti.

**Revendications**

1. Procédé de fabrication d'oléfines dans lequel on met un acide carboxylique ou un ester carboxylique en contact avec un catalyseur à une température de 200 à 400°, caractérisé en ce que le catalyseur renferme simultanément du nickel et au moins un métal du groupe formé par l'étain, le germanium et le plomb.

2. Procédé selon la revendication 1, caractérisé en ce que le catalyseur résulte de l'imprégnation de nickel de Raney ou de nickel supporté par une solution d'au moins un composé d'étain, de germanium et/ou de plomb.

3. Procédé selon la revendication 2, caractérisé en ce que le composé d'étain, de germanium et/ou de plomb est un hydrocarbyl étain, un hydrocarbyl germanium ou un hydrocarbyl plomb.

4. Procédé selon la revendication 3, caractérisé en ce que le composé d'étain est un alkyl étain.

5. Procédé selon la revendication 1, dans lequel le catalyseur résulte de l'imprégnation d'un support par une solution commune d'au moins un composé du nickel et au moins un composé d'étain, de germanium et/ou de plomb, ou de l'imprégnation d'un support par successivement une solution d'un composé de nickel et une solution d'un composé d'étain, de germanium et/ou de plomb.

6. Procédé selon la revendication 1, caractérisé en ce que le catalyseur contient en outre un composé à base de soufre, phosphore ou arsenic.

7. Procédé selon l'une des revendications 1 à 6, caractérisé en ce que le composé mis en contact avec le catalyseur est un acide ou un ester carboxylique linéaire.

**Patentansprüche**

1. Verfahren zur Herstellung von Olefinen, wobei man eine Carbonsäure oder einen Carbonsäureester in Kontakt mit einem Katalysator bei einer Temperatur von 200 bis 400°C bringt, dadurch gekennzeichnet, daß der Katalysator gleichzeitig Nickel und zumindest eines der Metalle Zinn, Germanium und Blei enthält.

2. Verfahren nach Anspruch 1, dadurch gekennzeichnet, daß der Katalysator durch Imprägnieren von Raney-Nickel oder Nickel auf einem Träger mit einer Lösung von zumindest einer Zinn-, Germanium- und/oder Bleiverbindung erhalten ist.

3. Verfahren nach Anspruch 2, dadurch gekennzeichnet, daß die Verbindung von Zinn, Germanium und/oder Blei eine Kohlenwasserstoffverbindung von Zinn, Germanium oder Blei ist.

4. Verfahren nach Anspruch 3, dadurch gekennzeichnet, daß die Zinnverbindung ein Zinnalkyl ist.

5. Verfahren nach Anspruch 1, dadurch gekennzeichnet, daß der Katalysator durch Imprägnierung eines Trägers mit einer gemeinsamen Lösung von zumindest einer Verbindung von Nickel und zumindest einer Verbindung von Zinn, Germanium und/oder Blei oder durch aufeinanderfolgendes Imprägnieren eines Trägers mit einer Lösung einer Nickelverbindung und einer Lösung einer Verbindung von Zinn, Germanium und/oder Blei erhalten ist.

6. Verfahren nach Anspruch 1, dadurch gekennzeichnet, daß der Katalysator außerdem eine Verbindung auf der Basis von Schwefel, Phosphor oder Arsen enthält.

7. Verfahren nach einem der Ansprüche 1 bis 6, dadurch gekennzeichnet, daß die in Kontakt mit dem Katalysator gebrachte Verbindung eine(e) geradkettige Carbonsäure oder Carbonsäureester ist.

**Claims**

1. A process for manufacturing olefins, comprising contacting a carboxylic acid or a carboxylic ester with a catalyst at a temperature from 200 to 400°C, characterized in that the catalyst simultaneously contains nickel and at least one metal from the group consisting of tin, germanium and lead.

2. A process according to claim 1, characterized in that the catalyst results from the impregnation of Raney nickel or of supported nickel with a solution of at least one tin, germanium and/or lead compound.

3. A process according to claim 1 or 2, characterized in that the tin, germanium and/or lead compound is a hydrocarbyl tin compound, a hydrocarbyl germanium compound or a hydrocarbyl lead compound.

4. A process according to claim 3, characterized in that the tin compound is an alkyl tin compound.

5. A process according to claim 1, wherein the catalyst results from the impregnation of a carrier by a common solution of at least one nickel compound and at least one tin, germanium and/or lead compound, or from the impregnation of a carrier, successively with a solution of a nickel compound and a solution of a tin, germanium and/or lead compound.

6. A process according to claim 1, characterized in that the catalyst further contains a sulfur, phosphorus or arsenic compound.

7. A process according to one of claims 1 to 6, characterized in that the compound which is contacted with the catalyst is a linear carboxylic acid or ester.